Europäisches Patentamt

European Patent Office

Office européen des brevets

(19)

(11) Veröffentlichungsnummer: **0 010 111**
**B1**

(12) # EUROPÄISCHE PATENTSCHRIFT

(45) Veröffentlichungstag der Patentschrift: **12.01.83**

(51) Int. Cl.³: **A 61 B 17/44**

(21) Anmeldenummer: **78101418.8**

(22) Anmeldetag: **21.11.78**

(54) Divergenz-Geburtszange.

(30) Priorität: **07.09.78 DE 7826632 U**

(43) Veröffentlichungstag der Anmeldung:
**30.04.80 Patentblatt 80/9**

(45) Bekanntmachung des Hinweises auf die Patenterteilung:
**12.01.83 Patentblatt 83/2**

(84) Benannte Vertragsstaaten:
**BE CH DE FR GB NL SE**

(56) Entgegenhaltungen:
**DE - A - 2 454 762**
**DE - C - 510 070**
**DE - C - 521 293**
**FR - A - 989 926**
**GB - A - 970 128**

(73) Patentinhaber: **Weyer, Klaus**
**Grossgrimberger Weg 8**
**D-5068 Odenthal (DE)**

(72) Erfinder: **Weyer, Klaus**
**Grossgrimberger Weg 8**
**D-5068 Odenthal (DE)**
Erfinder: **Quasthoff, Hans, Dr.**
**Bahnhofstrasse 29**
**D-5608 Radevormwald (DE)**

Courier Press, Leamington Spa, England.

Divergenz-Geburtszange

Die Erfindung betrifft eine Divergenz-Geburtszange, bestehend aus zwei lösbar miteinander verbundenen Zangengliedern.

Die gebräuchlichsten Geburtszangen sind entweder als Scherenzangen oder als Divergenzzangen ausgebildet und unterscheiden sich darüber hinaus im wesentlichen durch die Form der Löffel und die Arretiereinrichtung der beiden, durchweg um eine feste Achse drehbeweglich angeordneten Zangenglieder. Das ortsgebundene Gelenk besteht üblicherweise aus einer, an dem einen Zangenglied vorgesehenen Borrung, in welche ein an dem anderen Zangenglied befindlicher Zapfen eingreift. Da jedoch die beiden Zangenglieder beim Erfassen des kindlichen Kopfes unabhängig voneinander gehandhabt und sowohl in ihrer Längsrichtung als auch in Achsrichtung des Gelenkes im allgemeinen gegeneinander verschoben werden, ist es bei des bekannten Zangen schwierig oder gar unmöglich, die beiden Zangenglieder nach erfolgtem Anlegen an den kindlichen Kopf miteinander in Eingriff zu bringen, ohne dabei auf die Zangenglieder eine verhältnismäßig große Kraft ausüben zu müssen, die sich auf den Fötus schädigend auswirkt.

Die als Scherenzangen konzipierten Geburtszangen, bei denen sich die Zangenlöffel kreuzen, haben darüber hinaus den Nachteil, daß bei Zug- und/oder Druckbelastung der Zange die Zangellöffel in Schließrichtung, das heißt gegen den Kopf des Kindes bewegt werden, was die Gefahr in sich birgt, daß der Kopf einem unerwünscht hohen Druck ausgesetzt wird, falls es nicht gelingt, die Zange beim Anlegen an den Kopf rechtzeitig in einer angemessenen kopfschonenden Position zu arretieren, oder falls sich die Zange aus der bereits herbeigeführten Verschlußstellung unbeabsichtigt löst. Dieser Mangel tritt bei den Divergenz-Geburtszangen grundsätzlich nicht auf, weshalb sich diese in zunehmendem Maße in der Praxis durchgesetzt haben. Bei den Divergenzzangen verlaufen die Löffel parallel zueinander, so daß beim Betätigen der Zangengriffe die Löffel in Öffnungsrichtung bewegt werden, was einer Druckentlastung des kindlichen Kopfes entspricht. Allerdings besteht bein diesen Zangen die Gefahr, daß sie sich bei Zugbeanspruchung zu weit öffnen und deshalb vom Kopf abrutschen.

Diesen Mangel bei den bekannten, einen festen Drehpunkt für die beiden Zangenglieder aufweisenden Scheren- und Divergenz-Geburtszangen hat ZEPPELIN in seiner Offenlegungsschrift 24 52 762 u.a. durch Anordnen eines Schiebers über einer schiefen Ebene zwischen den Zangengriffen zu beseitigen versucht. Diese Lösung hat sich jedoch nicht bewährt, da sich der Schieber, der zwecks rascher Handhabung leichtgängig angebracht sein muß, in bestimmter Neigungsstellung der Zange selbsttätig verlagert, und zwar derart, daß sich die Zange entweder mit zu starkem Druck an den Kopf anlegt oder sich von diesem in unerwünschter Weise löst.

Schließlich haben alle Geburtszangen mit fester Drehachse noch den Nachteil, daß sich die Zangenglieder nicht in Längsrichtung verschieben lassen, wodurch sie insbesonders bei schwierigen Hinterkopf-, sowie bei Stießlagen unbrauchbar sind. Dieser Mangel ist bei der bekannten Zange nach KJELLAND dadurch behoben, daß das eine Zangenglied eine U-förmige, zur Längsinnenseite offene Lasche besitzt, in welcher das andere Zangenglied längsverschiebbar geführt ist. Dem dadurch erreichten Vorteil der axialen Verschiebbarkeit der beiden Zangenglieder, steht jedoch der Nachteil gegenüber, daß die Zangenglieder nicht arretiert werden könne, sondern von Hand in der erforderlichen gegenseitigen Zuordnung gehalten werden müssen, was bei der starken Zugbeanspruchung während der Geburt so gut wie nicht durchführbar ist. Im übrigen handelt es sich aus bei dieser Zange um eine Scherenzange mitt allen oben beschriebenen Nachteilen.

Eine weitere bekannte Zange mit axialer Verschiebbarkeit der Zangenlieder, ist die in der Patentschrift DE—C—521 293 beschriebene Geburtszange nach RODERICK. Hier bildet jedoch der Gleitschienen-mechanismus eine konstruktive Voraussetzung zur Erreichung des Hauptzweckes, nähmlich der Schwenkbarmachung der Zangenlöffel gegenüber den Schenkeln. Im übrigen ist diese Geburtszange in ihrer Handhabung so kompliziert und in Ihrer Funktion so unzuverlässig, daß sie heute kaum mehr bekannt ist und nur mehr historisches Interesse besitzt.

Der vorliegenden Erfindung liegt nun die Aufgabe zugrunde, eine Divergenz-Geburtszange zu schaffen, deren beide Zangenglieder sich nach erfolgtem Anlegen an den kindlichen Kopf möglichst rasch ohne Schwierigkeiten zusammenfügen und auf einfachste Weise in der gewünschten gegenseitigen Zuordnung zuverlässig arretieren lassen.

Diese Aufgabe wird erfindungsgemäß dadurch gelöst, daß jedes der beiden Zangenglieder zwischen Griff und Löffel einen als Gleitschiene ausgebildeten Mittelteil aufweist, wobei diese beiden Mittelteile in gleitenden Eingriff miteinander bringbar sind und der Mittelteil des einen Zangengliedes zwei gleitflächenseitig angeordnete Führungsteile besitzt, zwischen welchen der Mittelteil des anderen Zangengliedes verschiebbar geführt ist, und wobei an dem einen Führungsteil im Abstand zur Gleitfläche, sowie parallel zu dieser eine Arretierscheibe drehbar gelagert ist, mittels welcher die Mittelteile der beiden Zangenglieder festklemmbar sind.

Erfindungsgemäß ist die lichte Weite zwischen den beiden Führungsteilen des einen Zangengliedes größer als die Breite des Mittelteils des anderen Zangengliedes im Eingriffbereich.

Gemäß der Erfindung ist somit in vorteilhafter Weise erreicht, daß im Gegensatz zu den bekannten Divergenz-Geburtszangen, die eine ortsfeste Drehachse für die beiden Zangenglieder besitzen, zwischen den beiden Führungsteilen des einen Zangengliedes ein relativ großßer, über die Breite des einzulegenden anderen Zangengliedes hinausragender Abstand vorhanden ist, der bei in Öffnungsstellung befindlicher Arretierscheibe uneingeschränkt zur Verfügung steht und ein leicht zu handhabendes, störungsfreies Zusammenfügen der beiden Zangenglieder gestattet, und zwar auch dann, wenn diese wegen komplizierter Geburtslage des Kopfes, in Zangenlängsrichtung gegeneinander verschoben sind. Außerdem ist es als günstig anzusehen, daß die erfindungsgemäße Zange im Bereich zwischen den beiden Führungsteilen einen verlagerbaren Drehpunkt besitzt, welcher der jeweiligen Stellung der Zangenglieder anpaßber ist. Ein weiterer Vorteil dieser Zange besteht darin, daß sich die beiden Zangenglieder nach erfolgtem Anlegen an den Kopf, mittels der erfindungsgemäßen Arretierscheibe, in der gewünschten Position zuverlässig verriegeln lassen. Schließlich sind die Kurvenform und die exzentrische Lagerung der Arretierscheibe einerseits, und die Krummung des als Strutzlager dienenden Führungsteils andererseits so gewählt, daß bei jeder möglichen Zangeneinstellung die von der Zange auf den kindlichen Kopf einwirkende Anstellkraft, stets annähernd gleich groß ist. Demzufolge bietet die erfindungsgemäße Geburtszange die Gewähr dafür, daß ihre beiden Zangenglieder während der Geburt, das heißt für die Dauer der Zugbeanspruchung der Zange, die zuvor herbeigeführte optimale Relativstellung gegenüber dem kindlichen Kopf beibehalten.

Eine vorteilhafte Gestaltung der erfindungsgemäßen Zange besteht darin, daß einerseits die Außenflanke der am Mittelteil des einen Zangengliedes angeordneten Seitenwand und andererseits die Innenflanke des mit dieser Seitenwand in Anlage geratenden Führungsteils des anderen Zangengliedes, jeweils mit einer Reibfläche, beispielsweise in Form einer Verzahnung, versehen ist. Dadurch wird die von der druckbeaufschlagten Arretierscheibe auf die beiden Zangenglieder ohnehin ausgeübte Reibkraft zusätzlich erhöht und eine Verriegelung der Zangenglieder in Achs-, bzw. in Zugrichtung bewirkt.

Die Erfindung wird nachfolgend anhand eines Ausführungsbeispiels näher erläutert. In der Zeichnung zeigen:

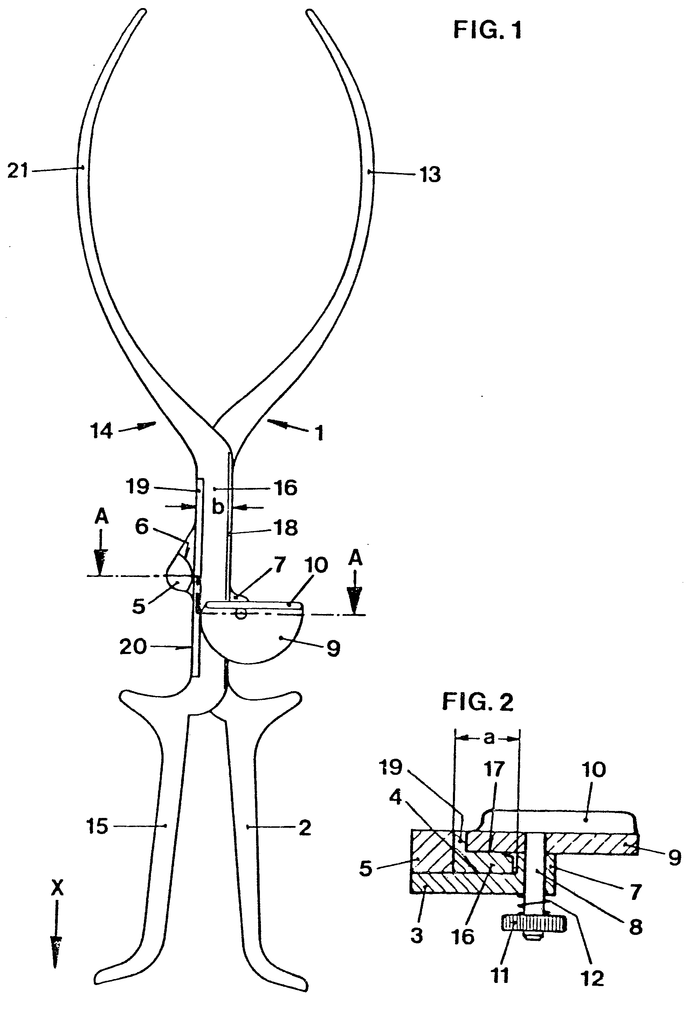

Fig. 1 eine erfindungsgemäße Geburtszange in größter Öffnungsstellung in Draufsicht und

Fig. 2 denselben Gegenstand im Schnitt nach der Linie A—A der Fig. 1 in vergrößerter Darstellung.

Die erfindungsgemäße Geburtszange besteht aus zwei gleich langen Zangengliedern 1 und 14, die sich im wesentlichen durch unterschiedlich ausgebildete Mittelteile 3 und 16 voneinander unterscheiden, in Größe und Form ihrer Griffe 2 und 15 und Löffel 13 und 21 jedoch übereinstimmen. Das Zangenglied 1 besitzt an seiner als Gleitfläche für das Zangenglied 14 dienenden Seite 4 des Mittelteils 3 zwei senkrecht angeordnete Führungsteile 5 und 7. Das Führungsteil 5 ist an seiner konvex gekrümmten Innenflanke 6 mit einer Reibfläche, beispielsweise in Form einer Zahnung, versehen. In dem Führungsteil 7 ist ein Bolzen 8 drehbar gelagert, an dessen einem freien Ende eine mit einer Handhabe 10 versehenen Arretierscheibe 9, und an dessen anderem freien Ende eine Scheibe 11 befestigt ist. Zwischen der Scheibe 11 und den Mittelteil 3 befindet sich eine Druckfeder 12, welche die Arretierscheibe 9 gegen das Führungsteil 7 drückt.

Das Mittelteil 16 des einen Zangengliedes 14 weist außenseitig eine Seitenwand 19 auf, die über die angrenzende Klemmfläche 17 hinausragt und mit dieser eine zur Zangeninnenseite offene Stufe bildet. Die Außenflanke 20 der Seitenwand 19 besitzt eine Reibfläche, bespielsweise in Form einen Zahnung. Das Mitteltiel 16 geht in seinem freien Längskantenbereich von der Klemmfläche 17 in eine Anlaufschräge 18 über.

Bei Benutzung der Zange wird zuerst das mit der Arretiereinrichtung versehene Zangenglied 1 und anschließend das Zangenglied 14 löffelseitig an den Kopf des Kindes angelegt. Im Folgenden wird das Zangenglied 14 mit dem Zangenglied 1 derart zusammengeführt, daß das Mittelteil 16 zwischen die beiden Führungsteile 5 und 7 gelangt. Die lichte Weite a zwischen den Führungsteilen 5 und 7 ist größer als die Breite b des Mittelteils 16. Beim Zusammenfügen spielt die gengeseiteige Zuordnung der Zangenglieder 1 und 14 in Längsrichtung keine Rolle. Die beiden Zangenglieder 1 und 14 sind in jeder möglichen axialen Verschiebestellung mit Hilfe der Arretierscheibe 9 feststellbar. Zum Verriegeln der beiden Zangenglieder 1 und 14 wird die Arretierscheibe 9 mittels ihrer Handhabe 10 im Uhrzeigersinn bewegt. Die Federbeaufschlagte Arretierscheibe 9 läuft dabei mit Ihrer Unterseite über die Anlaufschräge 18 auf die Klemmfläche 17 des Mittelteils 16 des Zangegliedes 14 und schließt so die Zange. Die Arretierscheibe 9 wird so weit gedreht, bis sie mit ihrer kurvenförmig verlaufenden Außenseite an die Seitenwand 19 stößt, die sich ihrerseits mit der Außenflanke 20 an der Innenflanke 6 des Führungsteiles 5 abstützt. Somit wird mit der Arretierscheibe 9 über das Mitteltiel 3 und 16 und die Löffel 13 und 21 eine definierte Anstellkraft auf den Kindlichen Kopf übertragen. Die in der beschriebenen Weise angelegte und arretierte Zange wird in der Folge in Zugrichtung X beansprucht. Ändert sich die Lage des kindlichen Kopfes im Geburtsverlauf, können die Löffel 21 und 13 jederzeit durch Lösen und erneutes Verschließen der Arretierscheibe 9 in der jeweilig sich ergebenden Kopflage neu fixiert werden um der Geburtsverlauf weiter optimal zu unterstützen.

**Patentansprüche**

1. Divergenz-Geburtszange, bestehend aus zwei lösbar miteinander verbundenen Zangengliedern (1,14), an deren jeweils entgegengesetzten Enden sich Griffe (2,15) und Löffel (13,21) befinden, wobei jedes der Zangenglieder (1,14) einen als Gleitschiene (3,4) ausgebildeten Mittelteil (3,16) aufweist, die in gleitenden Eingriff miteinander bringbar sind, wobei das eine Zangenglied (1) zwei Führungssteile (5,7) aufweist, dadurch gekennzeichnet, daß die Führungsteile (5,7) an den gegenüberliegenden Flanken des Mittelteiles (3) des Zangengliedes (1) in Achsrichtung des Mittelteiles (3) versetzt zueinander angeordnet sind, wobei sich an dem näher am Griff (2) gelegenen Führungsteil (7) eine drehbar gelagerte Arretierscheibe (9) befindet, deren Form so gewählt ist, daß sie in Öffnungsstellung die lichte Weite (a) zwischen den beiden Führungsteilen (5,7), die größes ist als dit Breite (6) des Mittelteils (16) des anderen Zangenglieds (17), freigibt und im geschlossenen Zustand sowohl den Mittelteil (16) führt, als auch über eine Seitenwand (19) den Mittelteil (16) gegen das Führungsteil (5) des Zangengliedes (17) klemmt, wobei der zwischen den Zangenlöffeln (13,21) befindliche kindliche Kopf das Widerlager bildet.

2. Divergenz-Geburtszange nach Anspruch 1, dadurch gekennzeichnet, daß die Arretierscheibe (9) eine der Anstellkraft der Zangenlöffel (13,21) zugeordnete Kurvenform aufweist.

3. Divergenz-Geburtszange nach einem oder mehreren vorausgegangenen Ansprüchen, dadurch gekennzeichnet, daß das Mittelteil (16) und die Arretierscheibe (9) eine Anlaufschräge (18) besitzen.

4. Divergenz-Geburtszange nach einem oder mehreren vorausgegangenen Ansprüchen, dadurch gekennzeichnet, daß die Führungsachse (8) der Arretierscheibe (9) axial beweglich ist und über eine Feder (12) auf die Rängelmutter (11) mit definierter Kraft einseitig gegen die Klemmfläche (17) des Mittelteils (16) drückt.

5. Divergenz-Geburtszange nach einem oder mehreren vorangegangenge Ansprüchen, dadurch gekennzeichnet, daß das Mittelteil (16) des einen Zangengliedes (14) eine als Reibfläche ausgebildete Außenflanke (20) aufweist, beispielsweise in Form einer Verzahnung.

6. Divergenz-Geburtszange nach einem oder mehreren vorausgegangenen Ansprüchen, dadurch gekennzeichnet, daß die Innenflanke (6) des Rührungsteiles (5) kurvenförmig verläuft.

7. Divergenz-Geburtszange nach einem oder mehreren vorausgegangenen Ansprüchen, dadurch gekennzeichnet, daß die Innenflanke (6) des einen Führungsteiles (5) als Reibfläche, beispielsweise in Form einer Verzahnung, ausgebildet ist.

**Claims**

1. Diverging forceps, consisting of two arms (1,14) detachable connected with each other, each arm having a blade (13,21) at one end and a grip (2,15) on the other end, each arm having a middle section (3,16) constructed as a slide bar (3,4) which enables a sliding gearing, with one arm (1) having two guiding parts (5,7), characterized by, the guiding parts (5,7) at the opposite flanks of the middle section (3) of one arm (1) being staggered in the axial direction of the middle section (3) wherein the guiding part (7) situated the most closed to the grip has a rotatable locking disk (9) which has such a choosen form that it releases, in opening position, the inside width (a) between both of the guiding parts (5,7) which is larger as the width (b) of the middle section (16) of the other arm (14) and in closed position it guides the middle section (16) as well as it clamps by means of one side wall (19) the middle section (16) against the guide part (5) of the forceps arm (7) with the foetal head between the forceps blades (13,21) forming the support.

2. Diverging forceps as claimed in Claim 1, wherein, the locking disk (9) has a curved form associated with the supporting force of the forceps blades (13,21).

3. Diverging forceps as claimed in one or more previous claims, wherein, the middle section (16) and the locking disk (9) have a faced leading edge.

4. Diverging forceps as claimed in one or more previous claims, wherein, the pin guide (8) of the locking disk (9) is axially movable and by means of a spring (12) on a knurled nut (11) is pressing single sided with a defined force against the clamping surface (17) of the middle section (16).

5. Diverging forceps as claimed in one or more previous claims, wherein, the middle section (16) of one forceps arm (14) has a outer flank designed as a friction surface, for instance as a surface joggled with teeth.

6. Diverging forceps as claimed in one or more previous claims, wherein, the inner flank (6) of one of the guiding parts (5) has a curved shape.

7. Diverging forceps as claimed in one or more previous claims, wherein, the inner flank (6) of one of the guiding parts (5) is designed as a friction surface, for instance as a surface joggled with teeth.

## Revendications

1. La revendication de brevet concerne un forceps à divergence possédant deux branches unies (1,14) et démontable. Aux deux extrémités opposées de chaque branche se trouvent un manche (2,15) et une cuillère (13,21). Chaque branche (1,14) possède dans sa partie médiane (3,16) une forme de rail (3,4) correspondant à celle du rail de la branche (1,14) opposée, dont cependent l'une des branches (1) possède deux guides (5,7) de telle façon que le rail de la branche (14) puisse glisser sur le rail de la branche (1) entre les guides (5,7) de cette dernière. Le forceps possède les particularités suivantes. Les guides (5,7) sont déportés longitudinalement dans le sens axial de part et d'autre des flancs opposée de la partie mediale (3) de la branche (1). Le guide (7) le plus proche du manche (2) possède un maneton arrêtoire (9) pivotant autour d'un axe (8). Le maneton arrêtoire (9) en position ouverte détermine une lumière de largeur (a) plus importante que la largeur (b) de la partie médiale (16) de la branche opposée (14). En position fermée le maneton arrêtoire (9) permet le guidage de la partie médiale (16) dans le sens longitudinal de la branche (17) et appui sur la surface lattérale (19) de telle façon que la partie médiale (16) soit bloquée contre le guide (5), la tête de l'enfant constituent le contre-effet.

2. Le forceps à divergence possède apprés la revendication 1, la particularité, que la forme du maneton arrêtoire (9) détermine une pression limitée au travers des cuillères (13,21) sur la tête de l'enfant.

3. Le forceps à divergence possède après une ou plusieurs revendications, la particularité, que le maneton arrêtoire (9) et la partie médiale (16) possèdent un méplat.

4. Le forceps à divergence possède après une ou plusieurs revendications, la particularité, que l'axe guide (8) du maneton arrêtoire (9) est mobile dans le sens axial et qu'un ressort exercant sa détente sur un écron molleté permet la pression du maneton arrêtoire (9) sur la surface (17).

5. Le forceps à divergence possède après une ou plusieurs revendications, la particularité, que la partie médiale (16) de la branche (14) possède une surface de glissement extérieur rugeuse, par exemple une dentelure.

6. Le forceps à divergence possède après une ou plusieurs revendications, la particularité, que le flanc interne (6) du guide (5) possède une forme curviligne.

7. Le forceps à divergence possède après une ou plusieurs revendications, la particularité, que le flanc interne (6) du guide (5) possède une surface de glissement regeuse, par exemple une dentelure.

0010111

FIG. 1

FIG. 2